# EUROPEAN PATENT SPECIFICATION

(11) **EP 3 363 915 B1**
(45) Date of publication and mention of the grant of the patent: **12.01.2022**
(21) Application number: 18151533.9
(22) Date of filing: 12.01.2018
(51) Int. Cl.: C12Q 1/6844, C12Q 1/70

(54) **NUCLEIC ACID SEQUENCES FOR TYPING DETECTION OF CUTANEOUS HUMAN PAPILLOMAVIRUSES AND USE THEREOF**
NUKLEINSÄURESEQUENZEN ZUR TYPISIERUNG VON KUTANEN MENSCHLICHEN PAPILLOMAVIREN UND VERWENDUNGEN DAVON
SONDES POUR LE TYPAGE DU VIRUS DU PAPILLOME HUMAIN CUTANE ET LEURS UTILIZATIONS

(30) Priority: 15.02.2017 CN 201710081809
(43) Date of publication of application: 22.08.2018
(73) Proprietor: The First Hospital Of China Medicial University, Liaoning 110001 (CN); Institute Of Process Engineering Chinese Acadamy Of Sciences, 100190 Beijing (CN); Zhongke Xinray (Suzhou) Biological Science Technologies Co., Ltd., 215152 Suzhou (CN)
(72) Inventor: GAO, Xinghua, Shenyang, 110001 (CN); DU, Yuguang, Beijing, 100190 (CN); GE, Ge, Shenyang, 110001 (CN); MAO, Rui, Beijing, 100190 (CN); QI, Ruiqun, Shenyang, 110001 (CN); SUN, Ming, Suzhou, 215152 (CN); CHEN, Hongduo, Shenyang, 110001 (CN)
(74) Representative: ZHAOffice SPRL

(56) References cited:
- WO-A1-2010/128724
- LEI Y J ET AL: "Molecular Epidemiological Study on Prevalence of Human Papillomaviruses in Patients with Common Warts in Beijing Area", BIOMEDICAL AND ENVIRONMENTAL SCIENCES, ELSEVIER, AMSTERDAM, NL, vol. 22, no. 1, 1 February 2009 (2009-02-01), pages 55-61, XP026076108, ISSN: 0895-3988, DOI: 10.1016/S0895-3988(09)60023-4 [retrieved on 2009-02-01]
- LEI ET AL: "Development of a multiplex PCR method for detecting and typing human papillomaviruses in verrucae vulgaris", JOURNAL OF VIROLOGICAL METHODS, ELSEVIER BV, NL, vol. 147, no. 1, 14 December 2007 (2007-12-14), pages 72-77, XP022390472, ISSN: 0166-0934, DOI: 10.1016/J.JVIROMET.2007.08.005

<110> First Affiliated Hospital of China Medical University, Institute of
   process Engineering, Chinese Academy of Science, and Zhongke Xinrui (Suzhou)
Biotechnology Co., Ltd.
<120> Nucleic acid sequences for typing detection of cutaneous human
   papillomaviruses (HPVs) and use thereof
<130> PEP5009CN17001
<160> 60
<210> 1
   <211> 18
   <212> DNA
   <213> Artificial sequence
<220>
   <223> HPV1 forward primer
<400> 1
   gctgtactcc tgcttcag 18
<210> 2
   <211> 19
   <212> DNA
   <213> Artificial sequence
<220>
   <223> HPV1 reverse primer
<400> 2
   tgcatgttgc ttgaacaac 19
<210> 3
   <211> 20
   <212> DNA
   <213> Artificial sequence
<220>
   <223> HPV2 forward primer
<400> 3
   ctgccagttt acaggatac c 20
<210> 4
   <211> 18
   <212> DNA
   <213> Artificial sequence
<220>
   <223> HPV2 reverse primer
<400> 4
   agacacggta ggcatagc 18
<210> 5
   <211> 20
   <212> DNA
   <213> Artificial sequence
<220>
   <223> HPV3 forward primer
<400> 5
   tgggttgtac cccacctatg 20
<210> 6
   <211> 18
   <212> DNA
   <213> Artificial sequence
<220>
   <223> HPV3 reverse primer
<400> 6
   gcaggtggtc tggcaaat 18
<210> 7
   <211> 20
   <212> DNA
   <213> Artificial sequence
<220>
   <223> HPV4 forward primer
<400> 7
   cctgcaatag gtgaacattg 20
<210> 8
   <211> 19
   <212> DNA
   <213> Artificial sequence
<220>
   <223> HPV4 reverse primer
<400> 8
   ggccatttac aaactgtgg 19
<210> 9
   <211> 24
   <212> DNA
   <213> Artificial sequence
<220>
   <223> HPV5 forward primer
<400> 9
   gatccaaatg tttattgtag gatg 24
<210> 10
   <211> 21
   <212> DNA
   <213> Artificial sequence
<220>
   <223> HPV5 reverse primer
<400> 10
   attgacgatg tctaaactga c 21
<210> 11
   <211> 20
   <212> DNA
   <213> Artificial sequence
<220>
   <223> HPV7 forward primer
<400> 11
   gagtgtttag agtacgcttg 20
<210> 12
   <211> 20
   <212> DNA
   <213> Artificial sequence
<220>
   <223> HPV7 reverse primer
<400> 12
   cagacgagtt ttccacatct 20
<210> 13
   <211> 21
   <212> DNA
   <213> Artificial sequence
<220>
   <223> HPV8 forward primer
<400> 13
   tgttttagca caaatcaatg c 21
<210> 14
   <211> 22
   <212> DNA
   <213> Artificial sequence
<220>
   <223> HPV8 reverse primer
<400> 14
   cattccagaa gttaaacttt gc 22
<210> 15
   <211> 18
   <212> DNA
   <213> Artificial sequence
<220>
   <223> HPV9 forward primer
<400> 15
   accgtttgct aacagtgg 18
<210> 16
   <211> 22
   <212> DNA
   <213> Artificial sequence
<220>
   <223> HPV9 reverse primer
<400> 16
   gcctatttca atacctctac ag 22
<210> 17
   <211> 21
   <212> DNA
   <213> Artificial sequence
<220>
   <223> HPV10 forward primer
<400> 17
   catattaaag agcaacggtg g 21
<210> 18
   <211> 19
   <212> DNA
   <213> Artificial sequence
<220>
   <223> HPV10 reverse primer
<400> 18
   tcagaaggaa cacacaagc 19
<210> 19
   <211> 21
   <212> DNA
   <213> Artificial sequence
<220>
   <223> HPV12 forward primer
<400> 19
   ctcaaataac tatgccacag g 21
<210> 20
   <211> 22
   <212> DNA
   <213> Artificial sequence
<220>
   <223> HPV12 reverse primer
<400> 20
   gtcaccatct tcaatgaaag tg 22
<210> 21
   <211> 21
   <212> DNA
   <213> Artificial sequence
<220>
   <223> HPV14 forward primer
<400> 21
   aggtatagaa ataggcagag g 21
<210> 22
   <211> 18
   <212> DNA
   <213> Artificial sequence
<220>
   <223> HPV14 reverse primer
<400> 22
   ttctacacat ggcaaggc 18
<210> 23
   <211> 21
   <212> DNA
   <213> Artificial sequence
<220>
   <223> HPV27 forward primer
<400> 23
   ccaataggtc tgatgttcct t 21
<210> 24
   <211> 19
   <212> DNA
   <213> Artificial sequence
<220>
   <223> HPV27 reverse primer
<400> 24
   ggtccgagat agtggtact 19
<210> 25
   <211> 18
   <212> DNA
   <213> Artificial sequence
<220>
   <223> HPV28 forward primer
<400> 25
   cacaacaggg agattgcc 18
<210> 26
   <211> 18
   <212> DNA
   <213> Artificial sequence
<220>
   <223> HPV28 reverse primer
<400> 26
   aacatgctgt cgccatac 18
<210> 27
   <211> 18
   <212> DNA
   <213> Artificial sequence
<220>
   <223> HPV29 forward primer
<400> 27
   acagagtctc aaccgttg 18
<210> 28
   <211> 19
   <212> DNA
   <213> Artificial sequence
<220>
   <223> HPV29 reverse primer
<400> 28
   cgtgtcttcc aagctagtg 19
<210> 29
   <211> 18
   <212> DNA
   <213> Artificial sequence
<220>
   <223> HPV41 forward primer
<400> 29
   tactttcctc catgctgc 18
<210> 30
   <211> 18
   <212> DNA
   <213> Artificial sequence
<220>
   <223> HPV41 reverse primer
<400> 30
   aacctcaat cccacgaat 18
<210> 31
   <211> 22
   <212> DNA
   <213> Artificial sequence
<220>
   <223> HPV48 forward primer
<400> 31
   gagactctgt cttctttttt gg 22
<210> 32
   <211> 19
   <212> DNA
   <213> Artificial sequence
<220>
   <223> HPV48 reverse primer
<400> 32
   cgtctaagcc aataaggcc 19
<210> 33
   <211> 18
   <212> DNA
   <213> Artificial sequence
<220>
   <223> HPV49 forward primer
<400> 33
   cctgcagcaa gtcaacag 18
<210> 34
   <211> 19
   <212> DNA
   <213> Artificial sequence
<220>
   <223> HPV49 reverse primer
<400> 34
   gccatccgta cttacacta 19
<210> 35
   <211> 24
   <212> DNA
   <213> Artificial sequence
<220>
   <223> HPV50 forward primer
<400> 35
   ggatgctgat atattagctc atct 24
<210> 36
   <211> 21
   <212> DNA
   <213> Artificial sequence
<220>
   <223> HPV50 reverse primer
<400> 36
   tttctgtaag gttgacattc c 21
<210> 37
   <211> 21
   <212> DNA
   <213> Artificial sequence
<220>
   <223> HPV57 forward primer
<400> 37
   ccggatgagc tatatgtcaa g 21
<210> 38
   <211> 20
   <212> DNA
   <213> Artificial sequence
<220>
   <223> HPV57 reverse primer
<400> 38
   acaaagagac atttgtgctg 20
<210> 39
   <211> 18
   <212> DNA
   <213> Artificial sequence
<220>
   <223> HPV63 forward primer
<400> 39
   ttcctaccca accgatca 18
<210> 40
   <211> 20
   <212> DNA
   <213> Artificial sequence
<220>
   <223> HPV63 reverse primer
<400> 40
   ttatctccaa aggcaaatcg 20
<210> 41
   <211> 20
   <212> DNA
   <213> Artificial sequence
<220>
   <223> HPV65 forward primer
<400> 41
   ccattggatg tagttgctac 20
<210> 42
   <211> 18
   <212> DNA
   <213> Artificial sequence
<220>
   <223> HPV65 reverse primer
<400> 42
   atcctgacct tcttgagc 18
<210> 43
   <211> 20
   <212> DNA
   <213> Artificial sequence
<220>
   <223> HPV75 forward primer
<400> 43
   ccttaaaatg gccaatgaca 20
<210> 44
   <211> 22
   <212> DNA
   <213> Artificial sequence
<220>
   <223> HPV75 reverse primer
<400> 44
   cgtgggaaca taaatagagt tg 22
<210> 45
   <211> 18
   <212> DNA
   <213> Artificial sequence
<220>
   <223> HPV76 forward primer
<400> 45
   tccttactgt aggccacc 18
<210> 46
   <211> 18
   <212> DNA
   <213> Artificial sequence
<220>
   <223> HPV76 reverse primer
<400> 46
   acctctacag gcccaaac 18
<210> 47
   <211> 18
   <212> DNA
   <213> Artificial sequence
<220>
   <223> HPV77 forward primer
<400> 47
   tactacccca ggagactg 18
<210> 48
   <211> 18
   <212> DNA
   <213> Artificial sequence
<220>
   <223> HPV77 reverse primer
<400> 48
   aaacagttgt tcccgacg 18
<210> 49
   <211> 20
   <212> DNA
   <213> Artificial sequence
<220>
   <223> HPV94 forward primer
<400> 49
   gacttcactg cattacagtt 20
<210> 50
   <211> 18
   <212> DNA
   <213> Artificial sequence
<220>
   <223> HPV94 reverse primer
<400> 50
   ccaacgtttt ggtcacca 18
<210> 51
   <211> 18
   <212> DNA
   <213> Artificial sequence
<220>
   <223> HPV95 forward primer
<400> 51
   ttcttctttg gccgaagg 18
<210> 52
   <211> 22
   <212> DNA
   <213> Artificial sequence
<220>
   <223> HPV95 reverse primer
<400> 52
   cggttaaaaa gctgagattc ac 22
<210> 53
   <211> 21
   <212> DNA
   <213> Artificial sequence
<220>
   <223> HPV115 forward primer
<400> 53
   acatacaaag gactgacatc t 21
<210> 54
   <211> 22
   <212> DNA
   <213> Artificial sequence
<220>
   <223> HPV115 reverse primer
<400> 54
   gtagtatcta ccaatgcaaa cc 22
<210> 55
   <211> 18
   <212> DNA
   <213> Artificial sequence
<220>
   <223> HPV117 forward primer
<400> 55
   ctagttctgt tggggacg 18
<210> 56
   <211> 18
   <212> DNA
   <213> Artificial sequence
<220>
   <223> HPV117 reverse primer
<400> 56
   ccaccacagt cacaaaca 18
<210> 57
   <211> 19
   <212> DNA
   <213> Artificial sequence
<220>
   <223> HPV125 forward primer
<400> 57
   cctgattatt tgggcatgg 19
<210> 58
   <211> 21
   <212> DNA
   <213> Artificial sequence
<220>
   <223> HPV125 reverse primer
<400> 58
   gtgtaggaca atatacagca c 21
<210> 59
   <211> 18
   <212> DNA
   <213> Artificial sequence
<220>
   <223> HPV160 forward primer
<400> 59
   taggcctcag tggtcatc 18
<210> 60
   <211> 18
   <212> DNA
   <213> Artificial sequence
<220>
   <223> HPV160 reverse primer
<400> 60
   caatcacctg acgtggat 18

## Description

### Technical Field

The present invention relates to the field of microbial pathogen detection, and in particular, to nucleic acid sequences for typing detection of cutaneous human papillomaviruses (HPVs) and use thereof.

### Background art

Human papillomaviruses (HPVs) belong to the papillomavirus A genus in the family papovaviridae and are spherical DNA viruses that can cause the proliferation of mucosal squamous cells on human skin, as manifested by common warts and genital warts (condyloma acuminatum), and other symptoms. With the sharp rise in the incidence of condyloma acuminatum among other sexually transmitted diseases and the increased incidence of cervical cancer, anal cancer and so on, HPV infection has received more and more attention.

HPVs are ubiquitous in the natural environment, and are closely related to human health. More and more HPV-related diseases are explored. At present, more than 200 types of HPV have been isolated, and different HPV types can lead to different clinical manifestations. Different types of HPV cause different diseases, the prognoses of these diseases are also different, and even the clinical manifestations of the same disease are also slightly different depending on the type of HPV infected. Detection of the specific type of HPV infected in the patient is of great significance in the screening and diagnosis and in the treatment and prognosis of the disease.

HPVs are associated with a variety of mucocutaneous diseases, including benign and malignant proliferative lesions. HPVs can cause several types of cutaneous diseases such as benign skin warts, solar keratosis (AKs) and non-melanoma skin cancer (NMSCs). The cutaneous HPV types are also frequently detected on healthy human skin. Among the cutaneous HPV infection, the most common disease is skin warts, including common warts, plantar warts and flat warts. Infections with different types of HPV may lead to the occurrence of different skin warts. The common warts are often caused by infection with HPV types 1, 2, and 4. HPV type 2 is the most common type causing common warts on the hands and feet of human. The flat warts are often caused by HPV3, 10, and 28. The skin warts caused by different types of HPV has slightly different clinical manifestations, such as different degrees of keratinization. HPV infection has significant geographical differences. By detecting the type of HPV infection in patients of local region in combination with the analysis of the therapeutic effect, a molecular epidemiological basis is provided for facilitating the implementation of personalized therapeutic regimens for skin warts. However, the vast majority of existing HPV testing methods target mucosal HPVs, and no mature method and nucleic acid sequence for detecting cutaneous HPVs that can be used in clinic are available.

International Patent Application WO 2010/128724 discloses a detection method of human papillomavirus for detecting the presence or absence of and type of HPV-1, 2, 3, 4, 27 and 57 which are the human papillomaviruses that cause mucocutaneous warts. Some primers are directed to the L1 region and some to the E2-L2 region.

In an article by Lee et al: "Development of a multiplex PCR method for detecting and typing human papillomaviruses in verrucae vulgaris", JOURNAL OF VIROLOGICAL METHODS, vol 147, no 1 (2007-12-14) Page 72-77 dicloses primer pairs for detecting HPV-1, 2, 27 and 57, some primers are directed to the L1 region and others to other regions.

In summary, there is an urgent need for developing a detection method that is practical, accurate, specific and sensitive, so as to meet the requirement for typing detection of cutaneous HPVs in clinic.

### Summary

In view of the above problems, the present invention provides a set of nucleic acid sequences for typing detection of cutaneous human papillomaviruses (HPVs) and use thereof. By using the nucleotide sequences, the types of cutaneous HPVs can be rapidly and accurately detected, thereby meeting the requirement for typing detection of cutaneous HPVs in clinic.

To achieve the above object, the set of nucleic acid sequences for typing detection of cutaneous HPVs provided in the present invention are specifically nucleic acid sequences of 30 primer pairs that are useful in the typing detection of 30 cutaneous HPVs.

The nucleic acid sequences of the primers are designed and screened as follows. The L1 fragment of each cutaneous HPV is aligned, and the portion with a high difference is selected and used to design a nucleic acid sequence of a primer. 3-10 pairs of candidate primers are designed for each type. Then, a large number of repeated PCR reactions are performed with the multiple pairs of candidate primers using artificially synthesized standard viral nucleic acids (see Table 1 for the Genbank Accession Nos.) and a large number of HPV infected clinical biological samples as templates. The obtained products are sequenced, and the specificity and sensitivity of amplification are validated. Finally, 30 primer pairs with good specificity and sensitivity are obtained, and the nucleotide sequences of which are set forth in SEQ ID Nos: 1 to 60.

The nucleotide sequences of the 30 primer pairs and corresponding HPV types detected are shown in Table 2.

**Table 1. Genbank Accession Nos. of standard viral nucleic acid sequence**

| **HPV type** | **Genbank Accession No** | **HPV type** | **Genbank Accession No** |
|---|---|---|---|
| **HPV001** | **A09292** | **HPV048** | **U31789** |
| **HPV002** | **EF117890** | **HPV049** | **NC_001591** |
| **HPV003** | **X74462** | **HPV050** | **U31790** |
| **HPV004** | **X70827** | **HPV057** | **X55965** |
| **HPV005** | **M17463** | **HPV063** | **X70828** |
| **HPV007** | **NC_001595** | **HPV065** | **X70829** |
| **HPV008** | **M12737** | **HPV075** | **Y15173** |
| **HPV009** | **X74464** | **HPV076** | **Y15174** |
| **HPV010** | **NC_001576** | **HPV077** | **Y15175** |
| **HPV012** | **X74466** | **HPV094** | **AJ620211** |
| **HPV014** | **X74467** | **HPV095** | **AJ620210** |
| **HPV027** | **NC_001584** | **HPV115** | **FJ947080** |
| **HPV028** | **U31783.** | **HPV117** | **CQ246950** |
| **HPV029** | **U31784** | **HPV125** | **FN547152** |
| **HPV041** | **X56147** | **HPV160** | **AB745694** |

**Table 2. Nucleotide sequences of 30 primer pairs for detecting the type of HPVs.**

| SEQ ID No | HPV type detected | Primer | Nucleotide sequence |
|---|---|---|---|
| SEQ ID No: 1 | HPV1 | Forward primer | GCTGTACTCCTGCTTCAG |
| SEQ ID No: 2 | | Reverse primer | TGCATGTTGCTTGAACAAC |
| SEQ ID No: 3 | HPV2 | Forward primer | CTGCCAGTTTACAGGATACC |
| SEQ ID No: 4 | | Reverse primer | AGACACGGTAGGCATAGC |
| SEQ ID No: 5 | HPV3 | Forward primer | TGGGTTGTACCCCACCTATG |
| SEQ ID No: 6 | | Reverse primer | GCAGGTGGTCTGGCAAAT |
| SEQ ID No: 7 | HPV4 | Forward primer | CCTGCAATAGGTGAACATTG |
| SEQ ID No: 8 | | Reverse primer | GGCCATTTACAAACTGTGG |
| SEQ ID No: 9 | HPV5 | Forward primer | GATCCAAATGTTTATTGTAGGATG |
| SEQ ID No: 10 | | Reverse primer | ATTGACGATGTCTAAACTGAC |
| SEQ ID No: 11 | HPV7 | Forward primer | GAGTGTTTAGAGTACGCTTG |
| SEQ ID No: 12 | | Reverse primer | CAGACGAGTTTTCCACATCT |
| SEQ ID No: 13 | HPV8 | Forward primer | TGTTTTAGCACAAATCAATGC |
| SEQ ID No: 14 | | Reverse primer | CATTCCAGAAGTTAAACTTTGC |
| SEQ ID No: 15 | HPV9 | Forward primer | ACCGTTTGCTAACAGTGG |
| SEQ ID No: 16 | | Reverse primer | GCCTATTTCAATACCTCTACAG |
| SEQ ID No: 17 | HPV10 | Forward primer | CATATTAAAGAGCAACGGTGG |
| SEQ ID No: 18 | | Reverse primer | TCAGAAGGAACACACAAGC |
| SEQ ID No: 19 | HPV12 | Forward primer | CTCAAATAACTATGCCACAGG |
| SEQ ID No: 20 | | Reverse primer | GTCACCATCTTCAATGAAAGTG |
| SEQ ID No: 21 | HPV14 | Forward primer | AGGTATAGAAATAGGCAGAGG |
| SEQ ID No: 22 | | Reverse primer | TTCTACACATGGCAAGGC |
| SEQ ID No: 23 | HPV27 | Forward primer | CCAATAGGTCTGATGTTCCTT |
| SEQ ID No: 24 | | Reverse primer | GGTCCGAGATAGTGGTACT |
| SEQ ID No: 25 | HPV28 | Forward primer | CACAACAGGGAGATTGCC |
| SEQ ID No: 26 | | Reverse primer | AACATGCTGTCGCCATAC |
| SEQ ID No: 27 | HPV29 | Forward primer | ACAGAGTCTCAACCGTTG |
| SEQ ID No: 28 | | Reverse primer | CGTGTCTTCCAAGCTAGTG |
| SEQ ID No: 29 | HPV41 | Forward primer | TACTTTCCTCCATGCTGC |
| SEQ ID No: 30 | | Reverse primer | AACCTCAATCCCACGAAT |
| SEQ ID No: 31 | HPV48 | Forward primer | GAGACTCTGTCTTCTTTTTTGG |
| SEQ ID No: 32 | | Reverse primer | CGTCTAAGCCAATAAGGCC |
| SEQ ID No: 33 | HPV49 | Forward primer | CCTGCAGCAAGTCAACAG |
| SEQ ID No: 34 | | Reverse primer | GCCATCCGTACTTACACTA |
| SEQ ID No: 35 | HPV50 | Forward primer | GGATGCTGATATATTAGCTCATCT |
| SEQ ID No: 36 | | Reverse primer | TTTCTGTAAGGTTGACATTCC |
| SEQ ID No: 37 | HPV57 | Forward primer | CCGGATGAGCTATATGTCAAG |
| SEQ ID No: 38 | | Reverse primer | ACAAAGAGACATTTGTGCTG |
| SEQ ID No: 39 | HPV63 | Forward primer | TTCCTACCCAACCGATCA |
| SEQ ID No: 40 | | Reverse primer | TTATCTCCAAAGGCAAATCG |
| SEQ ID No: 41 | HPV65 | Forward primer | CCATTGGATGTAGTTGCTAC |
| SEQ ID No: 42 | | Reverse primer | ATCCTGACCTTCTTGAGC |
| SEQ ID No: 43 | HPV75 | Forward primer | CCTTAAAATGGCCAATGACA |
| SEQ ID No: 44 | | Reverse primer | CGTGGGAACATAAATAGAGTTG |
| SEQ ID No: 45 | HPV76 | Forward primer | TCCTTACTGTAGGCCACC |
| SEQ ID No: 46 | | Reverse primer | ACCTCTACAGGCCCAAAC |
| SEQ ID No: 47 | HPV77 | Forward primer | TACTACCCCAGGAGACTG |
| SEQ ID No: 48 | | Reverse primer | AAACAGTTGTTCCCGACG |
| SEQ ID No: 49 | HPV94 | Forward primer | GACTTCACTGCATTACAGTT |
| SEQ ID No: 50 | | Reverse primer | CCAACGTTTTGGTCACCA |
| SEQ ID No: 51 | HPV95 | Forward primer | TTCTTCTTTGGCCGAAGG |
| SEQ ID No: 52 | | Reverse primer | CGGTTAAAAAGCTGAGATTCAC |
| SEQ ID No: 53 | HPV115 | Forward primer | ACATACAAAGGACTGACATCT |
| SEQ ID No: 54 | | Reverse primer | GTAGTATCTACCAATGCAAACC |
| SEQ ID No: 55 | HPV117 | Forward primer | CTAGTTCTGTTGGGGACG |
| SEQ ID No: 56 | | Reverse primer | CCACCACAGTCACAAACA |
| SEQ ID No: 57 | HPV125 | Forward primer | CCTGATTATTTGGGCATGG |
| SEQ ID No: 58 | | Reverse primer | GTGTAGGACAATATACAGCAC |
| SEQ ID No: 59 | HPV160 | Forward primer | TAGGCCTCAGTGGTCATC |
| SEQ ID No: 60 | | Reverse primer | CAATCACCTGACGTGGAT |

The 30 primer pairs can be used to prepare a diagnostic product for rapid detection of the types of cutaneous HPVs, for example, a kit.

To achieve the above object, the present invention further provides a kit for detecting the types of cutaneous HPVs, which specifically comprises the following components: all of the 30 primer pairs, SYBR Green I, dNTPs, *pfu* DNA polymerase, and 10X *pfu* Buffer.

The present invention has the following beneficial effects.

The set of nucleic acid sequences for typing detection of cutaneous human papillomaviruses (HPVs) provided in the present invention have high sensitivity and strong specificity, and can meet the requirement for typing detection of cutaneous HPVs in clinic. Because most of the cutaneous HPVs belong to the Beta genus, and a few belong to the Alpha, Gamma, Mu or Nu genus, the homology between the HPV L1 fragments of different genera is more than 60%, and the homology between some types of HPVs needing typing detection is even as high as 89%, causing a high difficulty in the typing detection of cutaneous HPVs. According to the prior art, it is difficult to screen nucleic acid sequences of primers with suitable specificity and sensitivity for the 30 types of HPVs mentioned in the present invention. However, primers with good specificity and sensitivity are obtained via screening in the present invention after a large number of experimental tests, thereby achieving the typing detection of cutaneous HPV in clinic. In order to obtain primers with suitable nucleotide sequences, more than two years of research efforts and nearly one million research funds are spent in designing, screening and sequencing nucleic acid sequences of primers, and validating the specificity and sensitivity of amplification. The results show that by using the series of nucleic acid sequences of the primers, test results with high accuracy and sensitivity are obtained.

In the present invention, 30 pairs of nucleic acid sequences for typing detection of cutaneous HPVs are constructed, and the types detected cover all types that are needed in the clinic, thereby filling a gap in the method for typing detection of cutaneous HPV in clinical application.

### Brief Description of the Drawings

Fig. 1 shows primer screening curves.
Fig. 2 shows reaction curves for the sensitivity and specificity detection of primers.

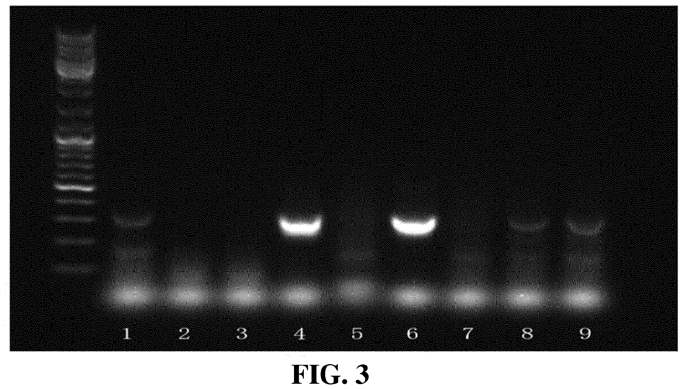

Fig. 3 shows electrophoresis of PCR products, in which Lane 1: Sample 1; Lane 2: Sample 2; Lane 3: Sample 3; Lane 4: Sample 4; Lane 5: Sample 5; Lane 6: Sample 6; Lane 7: Sample 7; Lane 8: Sample 8; and Lane 9: Sample 9.

### Detailed Description

The present invention will be further described with reference to the following specific examples, which are intended to facilitate the understanding of the present invention. However, these examples are merely provided for the purpose of illustrating the present invention, and the present invention is not limited thereto. The operations and methods that are not particularly described in the examples are all conventional operations and methods in the art.

### Example 1

Design and screening of nucleic acid sequences of the primers: The L1 fragment of each cutaneous HPV was aligned, and the portion with a high difference was selected and used to design a nucleic acid sequence of a primer. Multiple pairs of candidate primers are designed for each type. Then, a large number of repeated PCR reactions were performed with the multiple pairs of candidate primers using artificially synthesized standard viral nucleic acids and a large number of HPV infected clinical biological samples as templates. The obtained products were sequenced, and the specificity and sensitivity of amplification were validated. Finally, 30 primer pairs with good specificity and sensitivity were obtained, and the nucleotide sequences of which were set forth in SEQ ID Nos: 1 to 60.

Specifically, the screening method comprised the following steps.
(1) One candidate primer pair was randomly selected and subjected to an amplification reaction in a reaction system of 25 µl in a centrifuge tube. The reaction system comprised specifically each 0.2 µM of a forward primer and a reverse primer, SYBR Green I, 0.8 µM of dNTP, 2.5 U of *pfu* DNA polymerase, 5 µl of 10X *pfu* Buffer, and 1 ng/µl of plasmid DNA. Meanwhile, a negative control group was set, in which the components were the same as those in the above reaction system, except that the plasmid DNA was replaced by ddH₂O. The centrifuge tube was placed in an ABI Step One real time PCR Instrument, pre-denatured for 5 min at a temperature set to 94°C, and then subjected to 40 cycles of 50 s at 94°C, 50 s at 49°C, and 1 min at 72°C, followed by 5 min at 72°C. The resulting amplification curve or agarose gel electrophoretogram was observed to examine whether a desirable product exists. The HPV type in the sample was known, and thus primers having a nucleotide sequence set forth in SEQ ID No: 1 to 60 were preliminarily screened out by using the method.
(2) Detection of sensitivity and specificity of the screened primer pairs for 30 HPV types: The nucleic acid sequences of a primer pair for one HPV type were respectively reacted with various concentrations of plasmid DNA of this type and the plasmid DNAs of other HPV types. The reaction rate and the presence of cross-reactions were observed. The primer pair for each HPV type were examined with 8 groups of reaction system that was the same as the reaction system in the step 1, where 1 µL of various concentrations of plasmid DNA (1 ng/µl, 10-fold dilution of the plasmid DNA, and 2²⁰-fold dilution of the plasmid DNA) of this type was respectively added to 3 groups of reaction system, 1 ng/µl plasmid DNA of other HPV types was added respectively to 4 groups of reaction system, and ddH₂O was added in place of the plasmid DNA to 1 group of reaction system as a negative control group. The reaction was carried out in a fluorescence PCR instrument, amplification was observed, and the best primer pair with which the plasmid DNA at various concentrations of this type could be amplified without any cross-reaction, that is, the primer pair with high specificity and sensitivity, was screened out. Therefore, the nucleic acid sequences of the primers for 30 HPV types were ensured to have a high amplification efficiency while no cross-reactions exist.

### Example 2

### Detection method with nucleic acid sequences of candidate primer pair for detecting HPV type 1

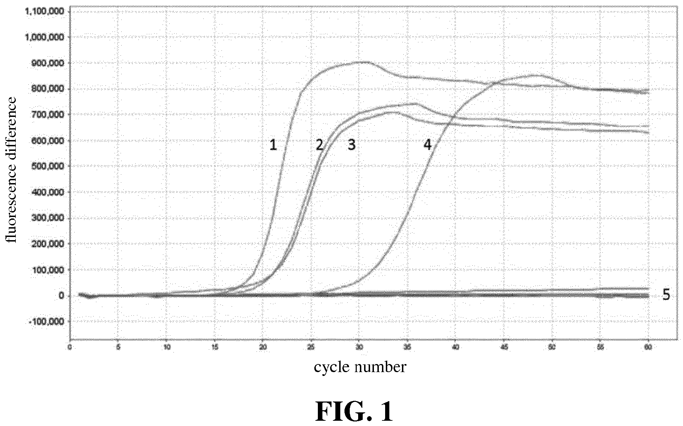

(1) One candidate primer pair for HPV type 1 was randomly selected, and subjected to an amplification reaction in a reaction system of 25 µl in a centrifuge tube. The reaction system comprised specifically each 0.2 µM of a forward primer and a reverse primer, SYBR Green I, 0.8 µM of dNTP, 2.5 U of *pfu* DNA polymerase, 5 µl of 10X *pfu* Buffer, and 1 ng/µl of plasmid DNA. Meanwhile, a negative control group was set, in which the components were the same as those in the above reaction system, except that the plasmid DNA was replaced by ddH₂O. The centrifuge tube was placed in a fluorescence PCR instrument for reacting, pre-denatured for 5 min at a temperature set to 94°C, and then subjected to 40 cycles of 50 s at 94°C, 50 s at 49°C, and 1 min at 72°C, followed by 5 min at 72°C. Then, 3 candidate primer pairs were additionally selected, with which the amplification reaction was repeated. The resulting amplification and primer screening curve are shown in Fig. 1, in which curve 1: amplification curve with a first primer pair for HPV type 1; curve 2: amplification curve with a second primer pair for HPV type 1; curve 3: amplification curve with a third primer pair for HPV type 1; curve 4: amplification curve with a fourth primer pair for HPV type 1; and curve 5: negative control group without plasmid DNA. The type of HPV1 in the sample was known, and thus primers having a nucleotide sequence set forth in SEQ ID No: 1 and SEQ ID No: 2 were screened out.
(2) Detection of sensitivity and specificity of the screened primer pairs for HPV type 1: The nucleic acid sequences of a primer pair for one HPV type were respectively reacted with various concentrations of plasmid DNA of this type and the plasmid DNAs of other HPV types. The reaction rate and the presence of cross-reactions were observed. The primer pair for each HPV type were examined with 8 groups of reaction system that was the same as the reaction system in the step 1, where various concentrations of plasmid DNA (1 ng/µl, 10-fold dilution of the plasmid DNA, and 2²⁰-fold dilution of the plasmid DNA) of this type was respectively added to 3 groups of reaction system, 1 ng/µl of plasmid DNA of other HPV types was added respectively to 4 groups of reaction system, and ddH₂O was added in place of the plasmid DNA to 1 group of reaction system as a negative control group. The reaction was carried out in a fluorescence PCR instrument, the amplification was observed, and the best primer pair with which the plasmid DNA at various concentrations of this type could be amplified without any cross-reaction, that is, the primer pair with high specificity and sensitivity, was screened out. Therefore, the nucleic acid sequences of the primers for 30 HPV types were ensured to have a high amplification efficiency while no cross-reactions exist.

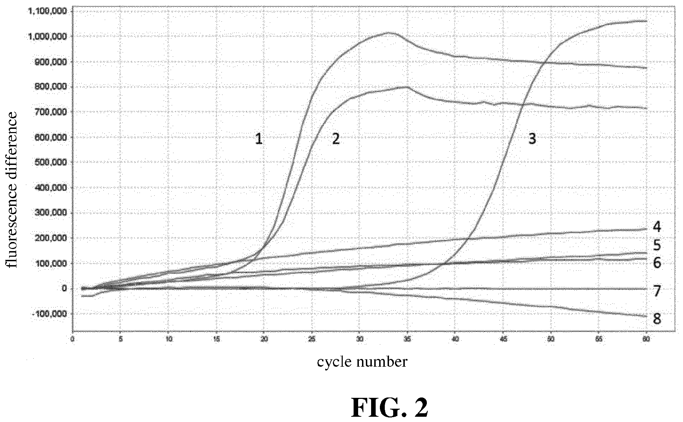

The reaction curves for detecting the sensitivity and specificity of the primer are shown in Fig. 2, in which curve 1: amplification curve with the primer sequence for HPV1 when the concentration of the HPV1 plasmid was 1 ng/µl; curve 2: amplification curve with the primer sequence for HPV1 when the concentration of the HPV1 plasmid DNA was 2-fold diluted; curve 3: amplification curve with the primer sequence for HPV1 when the concentration of the HPV1 plasmid was 2²⁰-fold diluted; curve 4: amplification curve with the primer sequence for HPV1 when HPV2, HPV3, HPV4, HPV5, HPV7, HPV8, and HPV9 plasmid DNAs were added; curve 5: amplification curve with the primer sequence for HPV1 when HPV10, HPV12, HPV14, HPV27, HPV28, HPV29, and HPV41 plasmid DNAs were added; curve 6: amplification curve with the primer sequence for HPV1 when HPV48, HPV49, HPV50, HPV57, HPV63, HPV65, and HPV75 plasmid DNAs were added; curve 7: amplification curve with the primer sequence for HPV1 when HPV76, HPV77, HPV94, HPV95, HPV115, HPV117, HPV125, and HPV160 plasmid DNAs were added; and curve 8: negative control group without plasmid DNAs.

### I. Detection of the HPV type in clinical sample

### Use of the nucleic acid sequences of the screened primer pair in the detection of the HPV type in clinical samples of skin warts

The samples were derived from dermatological outpatients with skin warts from the First Affiliated Hospital of China Medical University, and 30 clinical samples of skin warts were collected. DNA was extracted from the sample using a plasmid DNA Mini Extraction Kit, and about 1 ng of clinical sample DNA was added to a reaction system that was the same as the reaction system in the step 1 of Example 1. The sample was sequenced by using nucleic acid sequences of a universal primer pair for cutaneous HPVs, as shown in 3, in which Lane 1: electrophoretic band of the amplification product of Sample 1; Lane 2: electrophoretic band of the amplification product of Sample 2; Lane 3: electrophoretic band of the amplification product of Sample 3; Lane 4: electrophoretic band of the amplification product of Sample 4; Lane 5: electrophoretic band of the amplification product of Sample 5; Lane 6: electrophoretic band of the amplification product of Sample 6; Lane 7: electrophoretic band of the amplification product of Sample 7; Lane 8: electrophoretic band of the amplification product of Sample 8; and Lane 9: electrophoretic band of the amplification product of Sample 9. As can be seen from the comparison result of the HPV types detected in the samples by the two detection methods, the detection accuracy with the nucleic acid sequence of the present invention was 100%, and the sensitivity was higher. The detection results are shown in Table 3.

**Table 3. Comparison of the detection results of the two methods**

| **Clinical sample No.** | **Detection with the present nucleic acid sequence** | **Detection with a universal primer pair** | **Clinical sample No.** | **Detection with the present nucleic acid sequence** | **Detection with a universal primer pair** |
|---|---|---|---|---|---|
| **1** | **HPV27** | **HPV27** | **16** | **HPV2** | **HPV2** |
| **2** | **HPV27** | **Not detected** | **17** | **HPV57** | **HPV57** |
| **3** | **HPV27** | **Not detected** | **18** | **HPV1** | **Not detected** |
| **4** | **HPV1** | **HPV1** | **19** | **HPV27** | **Not detected** |
| **5** | **HPV27** | **Not detected** | **20** | **HPV27** | **HPV27** |
| **6** | **HPV27** | **HPV27** | **21** | **HPV57** | **HPV57** |
| **7** | **HPV1** | **Not detected** | **22** | **HPV1** | **HPV1** |
| **8** | **HPV27** | **HPV27** | **23** | **HPV1** | **HPV1** |
| **9** | **HPV57** | **HPV57** | **24** | **HPV4** | **Not detected** |
| **10** | **HPV57** | **HPV57** | **25** | **HPV27** | **Not detected** |
| **11** | **HPV1** | **HPV1** | **26** | **HPV27** | **HPV27** |
| **12** | **HPV2, HPV27** | **HPV27** | **27** | **HPV57** | **HPV57** |
| **13** | **HPV27** | **HPV27** | **28** | **HPV2** | **Not detected** |
| 14 | HPV57 | HPV57 | 29 | HPV2 | Not detected |
| 15 | HPV27 | Not detected | 30 | HPV27 | HPV27 |

The detection results show that the detection results with the nucleic acid sequences of the present invention are more accurate than the detection results obtained with a universal primer pair.
## Claims

1. A set of nucleic acid sequences for typing detection of cutaneous human papillomaviruses (HPVs), wherein the nucleic acid sequences are specifically nucleic acid sequences of 30 primer pairs that are useful in the typing detection of 30 cutaneous HPVs, and the nucleic acid sequences of the 30 primer pairs are set forth in SEQ ID NOs : 1 to 60.

2. A diagnostic product for rapidly detecting the types of cutaneous HPVs , wherein the diagnostic product is a kit comprising the set of 30 primer pairs according to claim 1.

3. The kit according to claim 2, wherein the kit specifically comprises the following components: all of the 30 primer pairs, SYBR Green I, dNTPs, *pfu* DNA polymerase, and 10X *pfu* Buffer.

4. The kit comprising 30 primer pairs according to claim 3, wherein the components in the kit are specifically 0.2 µM of each primer in the 30 primer pairs, 120 µM of SYBR, 0.8 µM of dNTP, 2.5 U of *pfu* DNA polymerase, and 5 µl of 10X *pfu* Buffer.

## Patentansprüche

1. Satz von Nukleinesäuresequenzen zur Typisierung von kutanen menschlichen Papillomaviren (HPVs), wobei die Nukleinesäuresequenzen spezifisch Nukleinesäuresequenzen von 30 Primerpaaren sind, die für die Typenerkennung von 30 kutanen HPVs nützlich sind, und die Nukleinesäuresequenzen der 30 Primerpaaren in den SEQ ID NOs : 1 bis 60 dargelegt sind.

2. Diagnostika-Produkt zur schnellen Erkennung der Typen von kutanen HPVs, wobei das Diagnostika-Produkt ein Kit ist, der den Satz von 30 Primerpaaren nach Anspruch 1 umfasst.

3. Kit nach Anspruch 2, wobei der Kit spezifisch die folgenden Komponenten umfasst: alle 30 Primerpaaren, SYBR Green I, dNTPs, Pfu-DNA-Polymerase und 10X-Pfu-Puffer.

4. Kit umfassend 30 Primerpaaren nach Anspruch 3, wobei die Komponenten im Kit spezifisch 0,2 µM jedes Primers aus den 30 Primerpaaren, 120 µM SYBR, 0,8 µM dNTP, 2,5 Einheiten Pfu-DNA-Polymerase, und 5 µl 10X-Pfu-Puffer umfassen.

## Revendications

1. Jeu de sondes nucléotidiques pour le typage de virus cutanés du papillome humain (HPVs), dans lequel les sondes nucléotidiques sont spécifiquement des séquences d'acides nucléiques de 30 paires d'amorces qui sont utiles pour la détection du typage de 30 HPVs cutanés, et les séquences d'acides nucléiques des 30 paires d'amorces sont énoncées dans SEQ ID NOs : 1 à 60.

2. Produit diagnostique pour la détection rapide des types d'HPV cutanés, dans lequel le produit diagnostique est un kit comprenant le jeu de 30 paires d'amorces selon la revendication 1.

3. Kit selon la revendication 2, dans lequel le kit comprend les composants suivants: toutes les 30 paires d'amorces, du SYBR Green I, des dNTPs, de l'ADN polymérase Pfu, et du Tampon Pfu 10X.

4. Kit comprenant 30 paires d'amorces selon la revendication 3, dans lequel les composants dans le kit sont spécifiquement 0,2 µM de chaque amorce parmi les 30 paires d'amorces, 120 µM de SYBR, 0,8 µM de dNTP, 2,5 Unités d'ADN polymérase Pfu, et 5 µl de Tampon Pfu 10X.
